# FASCICULE DE BREVET EUROPEEN

(11) **EP 3 545 080 B1**
(45) Date de publication et mention de la délivrance du brevet: **13.11.2024**
(21) Numéro de dépôt: 17816914.0
(22) Date de dépôt: 23.11.2017
(51) Int. Cl.: C12M 1/00, C12M 1/12, C12N 5/074, C12N 5/00

(54) **MICROCOMPARTIMENT CELLULAIRE ET PROCÉDÉS DE PRÉPARATION**
ZELLULARES MIKROKOMPARTIMENT UND HERSTELLUNGSVERFAHREN
CELLULAR MICROCOMPARTMENT AND PREPARATION METHODS

(30) Priorité: 23.11.2016 FR 1661377
(43) Date de publication de la demande: 02.10.2019
(60) Demande divisionnaire: 22214749.8
(73) Titulaire: Université de Bordeaux, 33000 Bordeaux (FR); Centre National de la Recherche Scientifique, 75016 Paris (FR); Institut d'Optique Théorique et Appliquée, 91127 Palaiseau Cedex (FR)
(72) Inventeur: FEYEUX, Maxime, 33400 Talence (FR); ALESSANDRI, Kevin, 33000 Bordeaux (FR); NASSOY, Pierre, 33000 Bordeaux (FR); COGNET, Laurent, 33000 Bordeaux (FR); RECHER, Gaëlle, 33400 Talence (FR); BEZARD, Erwan, 33000 Bordeaux (FR)
(74) Mandataire: Aquinov
(86) Numéro de dépôt international: PCT/FR2017/053225
(87) Numéro de publication internationale: WO 2018/096277

(56) Documents cités:
- EP-A1- 2 489 779
- EP-A1- 3 147 346
- WO-A1-2015/178427
- WO-A1-2016/141137
- US-A1- 2012 121 548
- US-A1- 2015 132 847
- ASHIDA TOMOAKI ET AL: "Propagation of human iPS cells in alginate-based microcapsules prepared using reactions catalyzed by horseradish peroxidase and catalase", ARTIFICIAL CELLS, NANOMEDICINE AND BIOTECHNOLOGY, vol. 44, no. 6, 6 July 2015 (2015-07-06), US, pages 1406 - 1409, XP055927007, ISSN: 2169-1401, DOI: 10.3109/21691401.2015.1029631
- KEVIN ALESSANDRI ET AL: "A 3D printed microfluidic device for production of functionalized hydrogel microcapsules for culture and differentiation of human Neuronal Stem Cells (hNSC)", LAB ON A CHIP, vol. 16, no. 9, 1 January 2016 (2016-01-01), UK, pages 1593 - 1604, XP055393107, ISSN: 1473-0197, DOI: 10.1039/C6LC00133E
- IKEDA KAZUHIRO ET AL: "3D culture of mouse iPSCs in hydrogel core-shell microfibers", 2015 28TH IEEE INTERNATIONAL CONFERENCE ON MICRO ELECTRO MECHANICAL SYSTEMS (MEMS), IEEE, 18 January 2015 (2015-01-18), pages 463 - 464, XP032740996, DOI: 10.1109/MEMSYS.2015.7050990
- TANIGUCHI KENICHIRO ET AL: "Lumen Formation Is an Intrinsic Property of Isolated Human Pluripotent Stem Cells", STEM CELL REPORTS, vol. 5, no. 6, 1 December 2015 (2015-12-01), United States, pages 954 - 962, XP055927010, ISSN: 2213-6711, DOI: 10.1016/j.stemcr.2015.10.015
- HUNT NICOLA C ET AL: "3D culture of human pluripotent stem cells in RGD-alginate hydrogel improves retinal tissue development", ACTA BIOMATERIALIA, ELSEVIER, AMSTERDAM, NL, vol. 49, 5 November 2016 (2016-11-05), pages 329 - 343, XP029885863, ISSN: 1742-7061, DOI: 10.1016/J.ACTBIO.2016.11.016
- WEI SONG ET AL: "Engraftment of human induced pluripotent stem cell-derived hepatocytes in immunocompetent mice via 3D co-aggregation and encapsulation", SCIENTIFIC REPORTS, vol. 5, no. 1, 23 November 2015 (2015-11-23), XP055393023, DOI: 10.1038/srep16884
- KEVIN ALESSANDRI ET AL: "A 3D printed microfluidic device for production of functionalized hydrogel microcapsules for culture and differentiation of human Neuronal Stem Cells (hNSC)", LAB ON A CHIP, vol. 16, no. 9, 26 April 2016 (2016-04-26), pages 1593 - 1604, XP055393107, ISSN: 1473-0197, DOI: 10.1039/C6LC00133E
- ISHIHARA KEISUKE; RANGA ADRIAN; LUTOLF MATTHIAS P; TANAKA ELLY M; MEINHARDT ANDREA: "Reconstitution of a Patterned Neural Tube from Single Mouse Embryonic Stem Cells", ORGAN REGENERATION : 3D STEM CELL CULTURE & MANIPULATION IN: METHODS IN MOLECULAR BIOLOGY, vol. 1597, 31 March 2017 (2017-03-31), US, pages 43 - 55, XP009502769, ISSN: 1940-6029, ISBN: 978-1-4939-6947-0, DOI: 10.1007/978-1-4939-6949-4_4
- MANSOURI VAHID ET AL: "Collagen-alginate microspheres as a 3D culture system for mouse embryonic stem cells differentiation to primordial germ cells", BIOLOGICALS, ACADEMIC PRESS LTD., LONDON, GB, vol. 48, 5 May 2017 (2017-05-05), pages 114 - 120, XP085125932, ISSN: 1045-1056, DOI: 10.1016/J.BIOLOGICALS.2017.04.003

## Description

L'invention a trait à un microcompartiment cellulaire permettant de conserver le caractère pluripotent de cellules humaines. L'invention a également trait à un procédé de préparation permettant d'obtenir de tels compartiments de culture cellulaire en trois dimensions (3D).

Les cellules pluripotentes sont considérées comme une ressource cellulaire humaine importante, et leur culture revêt un intérêt croissant, notamment dans le domaine médical et pharmaceutique. Ainsi, l'obtention de cellules pluripotentes en grandes quantités permettrait de répondre aux nouveaux besoins formulés par l'industrie pharmaceutique qui tend à réduire l'utilisation des modèles animaux et à utiliser des modèles cellulaires plus pertinents que les nombreuses lignées cellulaires utilisées à l'heure actuelle. Les tests haut débit mis au point par les groupes pharmaceutiques utilisent déjà de grandes quantités de cellules pluripotentes humaines. De même, l'ingénierie des tissus et la thérapie cellulaire chez l'homme sont conditionnées par la disponibilité de quantités industrielles de cellules pluripotentes humaines.

Actuellement, la culture de cellules pluripotentes humaines se fait principalement dans un environnement à deux dimensions (2D), tel que sur des boîtes de pétri, très éloigné du milieu en 3D dans lequel les cellules évoluent normalement. La manipulation de ces cellules cultivées en 2D est souvent délicate, et nécessite notamment des étapes de purification, détachement enzymatique, etc. En outre, ces cellules sont difficiles à conserver et présentent un taux de survie très faible à la congélation. Or, la possibilité d'envoyer par transporteur classique des cultures de cellules pluripotentes congelées en quantités massives, et compatibles avec la culture liquide, représente un enjeu majeur tant pour les laboratoires de recherche que pour les industries pharmaceutiques.

Face à cet état de fait, des systèmes de culture en trois dimensions ont été développés, qui cherchent notamment à accroître le débit, l'efficacité et la qualité des systèmes de culture de cellules souches pluripotentes humaines.

Cependant, les systèmes de culture en 3D existants ne donnent pas entière satisfaction. On observe souvent des phénomènes de fusion non contrôlés donnant naissance à des agrégats cellulaires dont la taille (>200 µm de diamètre) rend la diffusion du milieu de culture insuffisante. Ainsi, au sein de tels systèmes de culture en 3D, la différenciation cellulaire est difficilement maîtrisée et/ou le taux de mort cellulaire est très important. De manière générale, le manque d'homogénéité des produits issus de la culture de cellules 3D, ainsi que le coût de telles techniques rendent cette technologie non compétitive comparativement à la culture 2D qui pourtant ne donne pas satisfaction.

Par exemple, Ashida et al. « Propagation of Human iPS cells in alginate-based microcapsules prepared using reactions catalyzed by horseradish peroxidase and catalase" Artificial cells, Nanomedicine and Biotechnology 2015 Early Online 1-4, décrit des agrégats de cellules dans un hydrogel. Toutefois la prolifération des cellules est très faible et la pluripotence n'est pas assurée dans la solution proposée dans cet article

Il existe donc un besoin de système de culture cellulaire en 3D permettant de fournir de grandes quantités de cellules pluripotentes dont le phénotype est maîtrisé, et qui puissent être utilisées aisément tant pour la recherche fondamentale qu'au niveau industriel.

### Résumé de l'invention

En travaillant sur le développement de microcompartiments cellulaires pour la culture de cellules en 3D, les inventeurs ont mis au point un système permettant une culture de masse en suspension liquide de cellules pluripotentes humaines tout en conservant leur phénotype. Les microcompartiments développés permettent de cultiver les cellules en milieu liquide, en utilisant les milieux classiquement utilisés en culture 2D, tout en protégeant les cellules et en contrôlant leur phénotype pour éviter les différenciations sauvages et maintenir la pluripotence. Plus précisément, les microcompartiments, ou capsules, mis au point par les inventeurs comprennent successivement, organisées de manière sensiblement homocentrique une coque en hydrogel, une couche de matrice extracellulaire et une ou plusieurs couches de cellules pluripotentes humaines entourant une lumière centrale. La coque en hydrogel des capsules selon l'invention, contrairement aux systèmes de culture existants, protège les cellules des contraintes mécaniques liées aux collisions ou fusions lors de la culture en suspension liquide. De manière particulièrement avantageuse, l'organisation en « cystes » des microcompartiments selon l'invention, permet leur congélation avec un taux de survie cellulaire important. Les cellules peuvent en outre être différenciées avant utilisation, directement au sein du microcompartiment, ou être utilisées au stade pluripotent, en culture 3D comme en culture 2D. Les inventeurs ont également développé des méthodes de préparation de tels microcompartiments cellulaires, garantissant l'obtention et le maintien de la forme en cyste, propice tant à la congélation qu'au contrôle du phénotype des cellules qu'ils contiennent.

L'invention a donc pour objet un microcompartiment cellulaire comprenant successivement, organisées autour d'une lumière :
- au moins une couche de cellules pluripotentes humaines ;
- une couche de matrice extracellulaire ;
- une couche externe en hydrogel,
ledit microcompartiment étant clos.

Avantageusement, du milieu de culture comble les espaces laissés entre les couches.

L'invention a également pour objet un procédé de préparation d'un microcompartiment cellulaire selon l'invention, comprenant les étapes consistant à
(a) incuber des cellules souches pluripotentes humaines dans un milieu de culture contenant un inhibiteur des voies RHO/ROCK, ;
(b) mélanger les cellules souches pluripotentes issues de l'étape (a) avec une matrice extracellulaire ;
(c) encapsuler le mélange de l'étape (b) dans une couche d'hydrogel ;
(d) cultiver les capsules obtenues à l'étape (c) dans un milieu de culture contenant un inhibiteur des voies RHO/ROCK ;
(e) rincer les capsules issues de l'étape (d), de manière à éliminer l'inhibiteur des voies RHO/ROCK ;
(f) cultiver les capsules issues de l'étape (e) pendant 3 à 20 jours, préférentiellement 5 à 10 jours, dans un milieu de culture dépourvu d'inhibiteur des voies RHO/ROCK, et optionnellement récupérer les microcompartiments cellulaires obtenus.

L'invention a aussi pour objet un procédé de préparation d'un microcompartiment cellulaire selon l'invention, comprenant les étapes consistant à
(a) mélanger des cellules différenciées humaines avec une matrice extracellulaire et des agents de reprogrammation cellulaire ;
(b) encapsuler le mélange de l'étape (a) dans une couche d'hydrogel ;
(c) cultiver les capsules issues de l'étape (b) pendant 10 à 40 j ours, et optionnellement récupérer les microcompartiments cellulaires obtenus.

### Brève description des figures

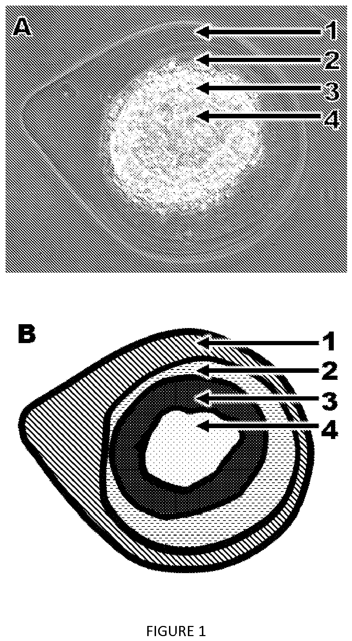

Figure 1 : Photo (A) et représentation schématique (B) d'un microcompartiment cellulaire formant un cyste selon l'invention (1 : couche d'hydrogel ; 2 : couche de matrice extracellulaire ; 3 : couches de cellules pluripotentes ; 4 : lumière).

### Description détaillée

L'invention a pour objet un microcompartiment cellulaire, en 3D, comprenant des cellules pluripotentes humaines, dans lequel le caractère pluripotent des cellules est conservé.

### Microcompartiment cellulaire

Le microcompartiment cellulaire selon l'invention forme un cyste, dont le centre creux, ou lumière, est préférentiellement aqueux. Dans le contexte de l'invention, un « cyste » s'entend d'une structure creuse fermée, contenant des couches sensiblement homocentriques, en ce sens qu'elles sont organisées successivement autour d'un même point, la couche externe enveloppant la couche de matrice qui enveloppe la couche de cellules, qui entoure la lumière. D'une manière générale, les cellules pluripotentes constituant le cyste sont polarisées La polarité de ces cellules à l'intérieur du cyste peut être mise en évidence par les protéines TJP-1 ou ZO-1, toutes deux localisées sur la face interne/apicale de la couche de cellules pluripotentes, qui jouxte la lumière.

La lumière est générée, au moment de la formation du cyste, par les cellules qui se multiplient et se développent sur la couche de matrice extracellulaire. Avantageusement, la lumière contient un liquide et plus particulièrement du milieu de culture.

Dans le contexte de l'invention, la « couche en hydrogel » désigne une structure tridimensionnelle formée à partir d'une matrice de chaînes de polymères gonflée par un liquide, et préférentiellement de l'eau. Avantageusement, l'hydrogel utilisé est biocompatible, en ce sens qu'il n'est pas toxique pour les cellules. En outre, la couche en hydrogel doit permettre la diffusion d'oxygène et de nutriments pour alimenter les cellules contenues dans le microcompartiment et permettre leur survie. Par exemple, la couche externe en hydrogel contient de l'alginate. Préférentiellement, la couche externe ne comprend que de l'alginate. Dans le contexte de l'invention, on entend par « alginate » des polysaccharides linéaires formés à partir de β-D-mannuronate (M) et α-L-guluronate (G), des sels et dérivés de ceux-ci.

Avantageusement, l'alginate est un alginate de sodium, composé à plus de 80% de G et moins de 20% de M, avec une masse moléculaire moyenne de 100 à 400 KDa (par exemple : PRONOVA^{®} SLG100) et une à concentration totale comprise entre 0.5% et 5% en masse. Selon l'invention, la couche en hydrogel est dépourvue de cellules. Dans un mode de réalisation du microcompartiment cellulaire selon l'invention, la couche externe comprend de l'alginate.

La couche de matrice extracellulaire peut quant à elle comporter quelques cellules. En effet, au moment de la formation du cyste, les cellules creusent leur espace dans la matrice et se multiplient, remplissant le microcompartiment. La délimitation entre la couche de matrice extracellulaire et la couche de cellules pluripotentes peut donc ne pas être parfaitement nette. Au niveau de la surface en contact avec la couche de cellules, la matrice extracellulaire peut ainsi contenir quelques cellules pluripotentes. A l'inverse, la surface de la couche de matrice extracellulaire en contact avec la couche d'hydrogel est exempte de cellules.

La couche de matrice extracellulaire est nécessaire à la survie des cellules pluripotentes dans le microcompartiment et à la création du cyste.

Préférentiellement, la couche de matrice extracellulaire forme un gel sur la face interne de la couche en hydrogel, c'est-à-dire la face dirigée vers la lumière du microcompartiment. La couche de matrice extracellulaire comprend un mélange de protéines et de composés extracellulaires nécessaires à la culture cellulaire, et plus particulièrement de cellules pluripotentes. Préférentiellement, la matrice extracellulaire comprend des protéines structurelles, telles que des laminines contenant les sous-unités α1, α4 ou α5, les sous-unités β1 ou β2, et les sous-unités γ1 ou γ3, de l'entactine, de la vitronectine, des laminines, du collagène, ainsi que des facteurs de croissance, tels que du TGF-béta et/ou de l'EGF. Dans un mode de réalisation, la couche de matrice extracellulaire consiste en, ou contient du Matrigel^{®} et/ou de la Geltrex^{®}.

Selon l'invention, le microcompartiment cellulaire contient une ou plusieurs couches de cellules souches pluripotentes humaines. Une cellule souche pluripotente, ou cellule pluripotente, s'entend d'une cellule qui a la capacité de former tous les tissus présents dans l'organisme d'origine entier, sans pour autant pouvoir former un organisme entier en tant que tel.

Dans un mode de réalisation particulier, les cellules encapsulées sont des cellules souches pluripotentes, telles que des cellules souches pluripotentes induites (IPS), des cellules MUSE (« Multilineage-differentiating Stress Enduring ») que l'on trouve dans la peau et la moelle osseuse des mammifères adultes, ou des cellules souches embryonnaires (ES) obtenues sans destruction de l'embryon dont elles sont issues.

Dans le contexte de l'invention, les « cellules souches pluripotentes induites » (CSPI ou IPS), s'entendent des cellules souches pluripotentes obtenues par reprogrammation génétique de cellules somatiques différenciées, et présentant une morphologie et un potentiel d'autorenouvellement et de pluripotence en partie similaires à ceux des cellules souches embryonnaires. Ces cellules sont notamment positives pour les marqueurs de pluripotence, comme la coloration à la phosphatase alcaline et l'expression des protéines NANOG, SOX2, OCT4 et SSEA3/4. Les procédés permettant l'obtention des cellules souches pluripotentes induites sont bien connus de l'homme du métier et sont notamment décrits dans les articles de Yu et al (Science, 2007, 318 (5858): 1917-1920), Takahashi et al (Cell, 2007, 131(5): 861-872) et Nakagawa et al (Nat Biotechnol, 2008, 26(1): 101-106).

Dans le cas de cellules souches embryonnaires obtenues sans destruction de l'embryon dont elles sont issues, lesdites cellules souches pluripotentes s'entendent des cellules dérivées de la masse cellulaire interne du blastocyste et qui ont la capacité de conduire à la formation de tous les tissus de l'organisme. La pluripotence des cellules souches embryonnaires peut être évaluée par la présence de marqueurs tels que les facteurs de transcription OCT4 et NANOG et des marqueurs de surface comme SSEA3/4, Tra-1-60 et Tra-1-81. Les cellules souches embryonnaires sont obtenues sans destruction de l'embryon dont elles sont issues, par exemple à l'aide de la technique décrite par Chung et al. (Cell Stem Cell, 2008, 2(2): 113-117).

Dans un mode de réalisation, les cellules souches pluripotentes humaines utilisées pour les microcompartiments selon l'invention sont induites à la pluripotence à partir de cellules somatiques.

Avantageusement, la couche de cellules contient au moins 95% en volume, préférentiellement au moins 96%, 97%, 98%, 99% de cellules et de matrice produite par lesdites cellules. Les cellules sont essentiellement des cellules pluripotentes. Par « essentiellement », on entend qu'au moins 90% des cellules contenues dans la couche de cellules sont des cellules pluripotentes, préférentiellement au moins 95%, 96%, 97%, 98%, 99%, 100%, sont des cellules pluripotentes.

Avantageusement, la lumière du cyste contient du milieu de culture. Notamment, tout milieu de culture permettant la culture en suspension de cellules pluripotentes peut être utilisé, et notamment tout milieu de culture classiquement utilisé en culture 2D.

Le microcompartiment cellulaire est clos. C'est la couche externe en hydrogel qui confère sa taille et sa forme au microcompartiment cellulaire. Le microcompartiment peut avoir n'importe quelle forme compatible avec l'encapsulation de cellules.

Avantageusement, les dimensions du microcompartiment cellulaire sont contrôlées. Dans un mode de réalisation, le microcompartiment cellulaire selon l'invention a une forme sphérique. Préférentiellement, le diamètre d'un tel microcompartiment est compris entre 10 µm et 1 mm, plus préférentiellement entre 50 µm et 500 µm, encore plus préférentiellement inférieur à 500 µm, de manière préférée inférieur à 400 µm.

Dans un autre mode de réalisation, le microcompartiment cellulaire selon l'invention a une forme allongée. Notamment, le microcompartiment peut avoir une forme ovoïde ou tubulaire. Avantageusement, la plus petite dimension d'un tel microcompartiment ovoïde ou tubulaire est comprise entre 10 µm et 1 mm, plus préférentiellement entre 50 µm et 500 µm, encore plus préférentiellement inférieure à 500 µm, de manière préférée inférieure à 400 µm. Par « plus petite dimension », on entend le double de la distance minimale entre un point situé sur la surface externe de la couche en hydrogel et le centre du microcompartiment.

Dans un mode de réalisation particulier, l'épaisseur de la couche externe en hydrogel représente 5 à 40% du rayon du microcompartiment. L'épaisseur de la couche de matrice extracellulaire représente 5 à 80 % du rayon du microcompartiment et est avantageusement accrochée sur la face interne de la coque en hydrogel. L'épaisseur de la couche de cellule pluripotentes représente environ 10% du rayon du microcompartiment. La couche de cellules pluripotente est en contact au moins en un point avec la couche de matrice extra cellulaire, un espace rempli par du milieu de culture peut être présent entre la couche de matrice et le cyste. La lumière représente alors de 5 à 30% du rayon du microcompartiment. Dans le contexte de l'invention, « l'épaisseur » d'une couche est la dimension de ladite couche s'étendant radialement par rapport au centre du microcompartiment.

Dans un exemple particulier, le microcompartiment cellulaire a une forme sphérique de rayon égal à 100µm. La couche en hydrogel a une épaisseur de 5µm à 40µm. La couche de matrice extracellulaire a une épaisseur de 5µm à environ 80µm. La couche de cellules pluripotentes a une épaisseur de 10 à 30 µm, la lumière ayant un rayon de 5 à 30 µm, environ.

D'une manière générale, la présence de la couche externe en hydrogel impose une taille maximale à la couche de cellules et permet, par confinement, de limiter la prolifération incontrôlée des cellules, qui pourrait entraîner la mort par anoxie des cellules et/ou des différenciations incontrôlées des cellules au niveau des couches les plus profondes, c'est-à-dire les plus proches de la lumière du cyste. En 2D, sur une boite de Pétri, les colonies sont des disques, les cellules au coeur du disque tendent à mourir (chaque nouvelle cellule issue d'une division est exclue de la colonie par l'absence de place) ou à se différencier sous les contraintes des cellules les entourant, les cellules du bord tendent à se différencier et seule une bande à la bonne distance présente le phénotype optimal. La topologie du microcompartiment présenté ici, la surface interne de la sphère formée par la capsule, permet de générer une « colonie » de cellules souches (la couche de cellules pluripotente) « sans bords » où toutes les cellules sont positionnées de manière optimale et équivalente tant pour la diffusion des petites molécules qu'en termes de contraintes mécaniques. Avantageusement, la densité cellulaire dans le microcompartiment est comprise entre 1 et plusieurs milliers de cellules par microcompartiment, préférentiellement entre 50 et 1000 cellules par microcompartiment de 100µm de rayon.

### Procédés de préparation de microcompartiments cellulaires

L'invention a également pour objet des procédés de préparation de microcompartiments cellulaires permettant d'obtenir le microcompartiment cellulaire selon l'invention. Plus particulièrement, l'invention propose de réaliser des microcompartiments cellulaires contenant des cellules souches pluripotentes organisées en cystes directement à partir de cellules souches pluripotentes, ou à partir de cellules différenciées qui seront reprogrammées en cellules pluripotentes à l'intérieur de la capsule d'hydrogel lors de la formation des microcompartiments.

Toute méthode de production de microcompartiments cellulaires contenant à l'intérieur d'une capsule d'hydrogel de la matrice extracellulaire et des cellules souches pluripotentes peut être utilisée pour la mise en oeuvre du procédé de préparation selon l'invention. Notamment, il est possible de préparer des microcompartiments en adaptant la méthode et le dispositif microfluidique décrits dans Alessandri et al., 2016 (« A 3D printed microfluidic device for production of functionalized hydrogel microcapsules for culture and differentiation of human Neuronal Stem Cells (hNSC) », Lab on a Chip, 2016, vol. 16, no. 9, p. 1593-1604), conformément aux étapes décrites ci-dessous.

Dans un premier mode de réalisation, le procédé de préparation selon l'invention comprend les étapes consistant à
(a) incuber des cellules souches pluripotentes humaines dans un milieu de culture contenant un inhibiteur des voies RHO/ROCK ;
(b) mélanger les cellules souches pluripotentes issues de l'étape (a) avec une matrice extracellulaire ;
(c) encapsuler le mélange de l'étape (b) dans une couche d'hydrogel ;
(d) cultiver les capsules obtenues à l'étape (c) dans un milieu de culture contenant un inhibiteur des voies RHO/ROCK ;
(e) rincer les capsules issues de l'étape (d), de manière à éliminer l'inhibiteur des voies RHO/ROCK ;
(f) cultiver les capsules issues de l'étape (e) pendant 3 à 20 jours, préférentiellement 5 à 10 jours, jusqu'à obtention d'un cyste, et optionnellement récupérer les microcompartiments cellulaires obtenus.

L'étape (a) d'incubation et l'étape (d) de culture dans un milieu contenant un ou plusieurs inhibiteurs des voies RHO/ROCK (« Rho-associated protein kinase »), tels que thiazovivin (C₁₅H₁₃N₅OS) et/ou Y-27632 (C₁₄H₂₁N₃O) permettent de promouvoir la survie des cellules souches pluripotentes, et l'adhérence des cellules à la matrice extracellulaire au moment de la formation de la couche externe d'hydrogel autour de ladite matrice extracellulaire. Il est toutefois souhaitable que ces étapes soient limitées dans le temps, afin que les inhibiteurs des voies RHO/ROCK n'empêchent pas la formation des cystes.

Ainsi, préférentiellement, l'incubation de l'étape (a) est conduite pendant un temps compris entre quelques minutes et quelques heures, préférentiellement entre 2 minutes et 2 heures, plus préférentiellement entre 10 minutes et 1 heure.

De même, préférentiellement, l'étape (d) de culture est conduite pendant un temps compris entre 2 et 48 heures, préférentiellement pendant un temps compris entre 6 et 24 heures, plus préférentiellement pendant un temps compris entre 12 et 18 heures.

L'étape (e) est nécessaire pour garantir l'élimination de toute trace d'inhibiteurs des voies RHO/ROCK. L'étape (e) est par exemple réalisée par rinçage, et préférentiellement par plusieurs rinçages, dans des milieux de culture successifs exempts d'inhibiteurs des voies RHO/ROCK.

Avantageusement, l'étape (f) est conduite pendant un temps suffisant pour obtenir un microcompartiment cellulaire dans lequel la couche de cellules pluripotentes et la lumière présentent une épaisseur cumulée égale à 10 à 95% du rayon du microcompartiment, soit pendant un temps suffisant pour permettre de passer de deux cellules à un millier de cellules environ. Tout milieu de culture adapté à la culture de cellules souches pluripotentes peut être utilisé, et notamment du tampon phosphate salin tel que le milieu « Roswell Park Memorial Institute medium ».

Dans un mode de mise en oeuvre, le procédé selon l'invention comprend une étape intermédiaire (a') consistant à dissocier les cellules souches pluripotentes issues de l'étape (a) avant l'étape (b), préférentiellement au moyen d'un réactif exempt d'enzyme. Avantageusement, ledit réactif est inhibé ou rincé avant l'étape d'encapsulation, notamment par rinçages successifs dans un milieu spécifique pour cellules pluripotentes. Par exemple, le réactif utilisé est un tampon iso-osmotique contenant de l'EDTA ou de l'EGTA tel que le ReLeSR^{®}. Bien entendu, il est également possible d'utiliser de la trypsine ou un réactif contenant une enzyme, mais le taux de survie des cellules pluripotentes à l'issue de cette étape peut alors être moindre comparativement à l'utilisation d'un réactif exempt d'enzyme. Dans tous les cas, l'étape de rinçage est nécessaire pour éliminer toute trace du réactif utilisé pour la dissociation des cellules.

Dans un mode de mise en oeuvre, au moins une des étapes (a'), (b), (c), (d) ou (e) est réalisée à une température comprise entre 0 et 8°C, préférentiellement l'ensemble des étapes (a'), (b), (c), (d) et (e). Le maintien d'une température sensiblement égale à 4°C permet de mettre les processus biologiques des cellules en dormance, y compris la transduction des signaux provenant de l'environnement extérieur. Il est ainsi possible de limiter le phénomène de mort cellulaire, qui pourrait être induit par le détachement cellulaire.

Dans un autre mode de réalisation, le procédé de préparation d'un microcompartiment cellulaire selon l'invention, comprend les étapes consistant à
(a) mélanger des cellules humaines différenciées avec une matrice extracellulaire et des agents de reprogrammation cellulaire non perméants vis-à-vis de la couche d'hydrogel ;
(b) encapsuler le mélange de l'étape (a) dans une couche d'hydrogel ;
(c) cultiver les capsules issues de l'étape (b) pendant 10 à 40 j ours, et optionnellement récupérer les microcompartiments cellulaires obtenus.

Dans un autre mode de réalisation, le procédé de préparation d'un microcompartiment cellulaire selon l'invention, comprend les étapes consistant à
(a) mélanger des cellules humaines différenciées avec une matrice extracellulaire ;
(b) encapsuler le mélange de l'étape (a) dans une couche d'hydrogel ;
(c) incuber les capsules issues de l'étape (b) avec agents de reprogrammation cellulaire perméants vis-à-vis de la couche d'hydrogel et cultiver les capsules pendant 10 à 40 jours, et optionnellement récupérer les microcompartiments cellulaires obtenus.

Par exemple, les cellules différenciées utilisées sont des fibroblastes.

L'homme du métier sait procéder à la reprogrammation d'une cellule différenciée en une cellule souche en réactivant l'expression des gènes associés au stade embryonnaire au moyen de facteurs spécifiques, désignés dans la présente invention comme « agents de reprogrammation ». A titre d'exemples, on peut citer les méthodes décrites dans Takahashi et al., 2006 (« Induction of pluripotent stem cells from mouse embryonic and adult fibroblast cultures by defined factors » Cell, 2006 Vol 126, pages 663-676), Ban et al., 2009 (« Efficient induction of transgene-free human pluripotent stem cells using a vector based on Sendai virus, an RNA virus that does not integrate into the host genome » Proc Jpn Acad Ser B Phys Biol Sci. 2009; 85(8):348-62) et dans la demande internationale WO2010/105311 ayant pour titre « Production of reprogrammed pluripotent cells ».

Les agents de reprogrammation sont avantageusement co-encapsulés avec les cellules différenciées, de manière à concentrer le produit et à favoriser le contact avec l'ensemble des cellules. Dans le cas d'agents de reprogrammation perméants à la couche d'hydrogel, il est possible d'ajouter lesdits agents dans le milieu de culture après l'étape d'encapsulation.

Les agents de reprogrammation permettent d'imposer aux cellules une succession de changements phénotypiques jusqu'au stade pluripotent. Avantageusement, l'étape (a) de reprogrammation est réalisée en utilisant des milieux de culture spécifiques, favorisant ces changements phénotypiques. Par exemple, les cellules sont mis en culture dans un premier milieu comprenant 10% de sérum humain, ou bovin, dans un milieu minimum essentiel de Eagle (DMEM) supplémenté avec un inhibiteur des récepteurs sérine/thréonine protéine kinase (tel que le produit SB-431542 (C₂₂H₁₆N₄O₃)), un ou plusieurs inhibiteurs des voies RHO/ROCK (« Rho-associated protein kinase »), tels que du thiazovivin et/ou Y-27632, des facteurs de croissance des fibroblastes, tel que du FGF-2, de l'acide ascorbique et des antibiotiques, tels que la Trichostatin A (C₁₇H₂₂N₂O₃). Puis le milieu de culture est remplacé par du milieu favorisant la multiplication des cellules pluripotentes, tel que le milieu mTeSR^{®}1.

Avantageusement, les capsules issues de l'étape (b) contiennent chacune entre 1 et 500 cellules différenciées, préférentiellement entre 50 et 200.

Dans un mode de mise en oeuvre, au moins une des étapes (a), (b), (c) ou (d) est réalisée à une température comprise entre 0 et 4°C, préférentiellement l'ensemble des étapes (a), (b), (c) et (d). Le maintien d'une température inférieure ou égale à 4°C permet de mettre les processus biologiques des cellules en dormance, y compris la transduction des signaux provenant de l'environnement extérieur. Il est ainsi possible de limiter le phénomène de mort cellulaire, qui pourrait être induit par le détachement cellulaire.

Les microcompartiments obtenus au cours de l'étape (d) peuvent être triés de manière à isoler les microcompartiments cellulaires présentant la forme en cyste souhaitée. Une telle étape de tri peut se faire en continu, de manière à séparer les microcompartiments cellulaires présentant déjà la forme en cyste souhaitée, des microcompartiments encore en cours de formation. Une telle étape de tri peut se faire par simple analyse morphologique, sans perturber les autres microcompartiments au sein desquels la reprogrammation est encore en cours et/ou l'organisation en cyste non encore achevée.

D'une manière générale, les microcompartiments cellulaires obtenus selon les procédés de l'invention peuvent ensuite être congelés avant toute utilisation. En effet, la forme en cyste favorise la survie des cellules au sein de microcompartiment, et après décongélation, le taux de survie est supérieur à 80%. Avantageusement, la congélation est réalisée en utilisant de l'azote liquide permettant de vitrifier rapidement les microcompartiments et de limiter les risques de formation de cristaux au sein des membranes lipidiques des cellules. Les microcompartiments cellulaires peuvent être mis en suspension dans un tampon de congélation favorisant la survie des cellules. Il est par exemple possible d'utiliser les tampons de congélation classiquement utilisés pour congeler les embryons.

Les microcompartiments cellulaires ainsi congelés peuvent ensuite être décongelés selon les besoins.

### Applications

Les microcompartiments cellulaires objets de la présente invention peuvent être utilisés pour de nombreuses applications. En effet, les cellules qu'ils renferment peuvent être aisément récupérées, par simple hydrolyse et/ou dissolution de la couche externe en hydrogel. En outre, il est possible de différencier les cellules pluripotentes à l'intérieur de la capsule d'hydrogel ou après hydrolyse/dissolution de ladite capsule d'hydrogel, selon les besoins, de manière à obtenir de grandes quantités de lignées cellulaires d'intérêt. Avantageusement, les cellules sont différenciées en un ou plusieurs types cellulaires d'intérêt, au sein du microcompartiment, c'est-à-dire avant hydrolyse de la couche externe en hydrogel.

Les microcompartiments cellulaires, et plus précisément les cellules qu'ils renferment peuvent être utilisés à des fins de recherche et développement, tant sous forme d'un réseau cellulaire 3D que plus classiquement en culture 2D. Il est également possible de les utiliser à des fins thérapeutiques, notamment pour des applications de thérapie cellulaire, ingénierie tissulaire, etc.

### EXEMPLES

### Exemple 1 : Protocole d'obtention de microcompartiments cellulaires à partir de cellules humaines induites à la pluripotence.

### Solutions utilisées :

Solution 1, base de milieu DMEMF12 supplémentée avec 2µM Thiazovivin
Solution 2, PBS sans magnésium et sans calcium supplémenté avec 1µM 2µM Thiazovivin
Solution 3, tampon de détachement cellulaire non-enzymatique : RelesR^{™} supplémenté avec 2µM Thiazovivin.
Solution 4, milieu de culture cellules souches pluripotentes : MTeSR1^{™} hES/hIPS cell medium STEMCELL^{™}).
Solution 4+, Solution 4 supplémentée avec 2µM Thiazovivin.
Solution 5, Matrigel^{™}.
Solution 6, sorbitol 300mM avec 2µM Thiazovivin.

### Solution cellulaire :

Une boite de Pétri de cellules IPS humaines (obtenues à partir de Primary Dermal Fibroblast; Normal, Human, Adult ATCC^{®} PCS-201-012^{™} et de CytoTune^{™}-iPS 2.0 Sendai Reprogramming Kit (ref A16517) en utilisant la technologie présentée dans l'exemple 2) de 25cm² à 90% de confluence est utilisée par la suite pour correspondre aux volumes recommandés. Toutes les étapes suivantes sont effectuées à 4°C jusqu'à la réticulation de la coque en hydrogel dans le bain de calcium.

Étape 1 : Rincer les cellules avec la solution 1. Attendre de 10 minutes à 1 heure.

Étape 2 : Rincer deux fois avec 4 mL de solution 2.

Étape 3 : Aspirer délicatement la solution.

Étape 4 : Incuber les cellules avec 4 mL de solution 3 pendant 5-10 minutes.

Étape 5 : Détacher les cellules avec 2mL de solution 4+ avec une pipette à cône large pour réduire le stress de cisaillement.

Étape 6 : Centrifuger la suspension de cellules à 360 g pendant 5 minutes.

Étape 7 : Aspirer le surnageant.

Étape 8 : Resuspendre avec 0.5 mL de solution 4+.

Étape 9 : Centrifuger à nouveau à 360g et aspirer le surnageant.

Étape 10 : Re-suspendre le culot de cellules dans 70µL de solution 5 et 100µL de solution 6 (le volume du culot devrait être de 30µL). La solution cellulaire est prête.

### Encapsulation :

Le dispositif d'encapsulation est préparé comme décrit dans Alessandri et al., 2016 (« A 3D printed microfluidic device for production of functionalized hydrogel microcapsules for culture and differentiation of human Neuronal Stem Cells (hNSC) », Lab on a Chip, 2016, vol. 16, no. 9, p. 1593-1604).

En résumé, les différentes parties du dispositif sont stérilisées (par autoclave) ; Les trois solutions nécessaires sont chargées sur trois pousse-seringues, i) solution d'alginate (PRONOVA^{®}SLG100 à 2% en masse dans de l'eau distillée), ii) solution intermédiaire (sorbitol à 300mM), iii) solution cellulaire (préparée à l'étape précédente) ; Les trois solutions sont co-injectées de manière concentrique grâce à un injecteur microfluidique qui permet de former un jet qui se fractionne en gouttes dont la couche extérieure est la solution d'alginate et le coeur la solution de cellules ; Ces gouttes sont collectées dans un bain de calcium (à 100mM) qui rigidifie la solution d'alginate pour former la coque.

Pour améliorer la mono-dispersité des microcompartiments cellulaires, l'alginate a été chargé avec un courant continu à +2KV. Un anneau à la masse de 2cm de diamètre est disposé à 500µm de la pointe dans le plan perpendiculaire à l'axe du jet sortant de l'injecteur microfluidique pour générer le champ électrique.

On note que dans ces conditions d'encapsulation, la couche de Matrigel^{®} se forme spontanément.

### Traitement après encapsulation :

Étape 1 : Les capsules sont récupérées avec un tamis cellulaire 40µm puis après rinçage avec la solution 1 elles sont stockées dans une flasque de 75cm² avec 20mL de solution 4+.

Étape 2 : La flasque est gardée pendant 12h à l'incubateur à 37°C et 5% de CO₂.

Étape 3 : Changer le milieu pour la solution 4 pour permettre la formation des cystes.

Étape 4 : Après 24 à 72h des cystes de quelques dizaines de cellules se forment dans les capsules. Les microcompartiments cellulaires sont matures après 5 à 10 jours.

### Exemple 2 : Protocole d'obtention de microcompartiments cellulaires à partir de fibroblastes humains.

### Solutions utilisées :

Solution 1, base de milieu DMEMF12
Solution 2, PBS sans magnésium sans calcium supplémenté
Solution 3, tampon de détachement cellulaire trypsin EDTA
Solution 4, milieu de culture fibroblastes : 10% de sérum humain dans une base de milieu DMEM
Solution 4+, Solution 4 supplémentée avec 2µM Thiazovivin.
Solution 5, Matrigel^{™}.
Solution 6, sorbitol 300mM avec 2µM Thiazovivin.

### Solution cellulaire :

Une boite de Pétri de fibroblastes humains (Primary Dermal Fibroblast; Normal, Human, Adult (ATCC^{®} PCS-201-012^{®}) de 25cm² à faible densité de confluence est utilisée par la suite pour correspondre aux volumes recommandés (1 à 2 millions de cellules). Toutes les étapes suivantes sont effectuées à 4°C jusqu'à la réticulation de la coque dans le bain de calcium.

Étape 1 : Rincer les cellules avec la solution 2.

Étape 2 : Aspirer délicatement la solution.

Étape 3 : Incuber les cellules avec 4 mL de solution 3 pendant 5-10 minutes.

Étape 4 : Détacher les cellules avec 2mL de solution 4+ avec une pipette à cône large pour réduire le stress de cisaillement.

Étape 6 : Centrifuger la suspension de cellules à 360 g pendant 5 minutes.

Étape 7 : Aspirer le surnageant.

Étape 8 : Resuspendre avec 0.5 mL de solution 4+.

Étape 9 : Centrifuger à nouveau à 360g et aspirer le surnageant.

Étape 10 : Re-suspendre le culot de cellules dans 90µL de solution 5 et 100µL de solution 6 (le volume du culot devrait être de 10µL).

Étape 11 : Ajouter 1/10 du contenu du kit « CytoTune^{®} -IPS 2.0 Sendai Reprogramming Kit » (contenant un virus de reprogrammation) prévu pour une plaque 6 puits. La solution cellulaire est prête.

### Encapsulation :

L'encapsulation est réalisée conformément au protocole de l'exemple 1.

### Traitement après encapsulation :

Étape 1 : Les capsules sont récupérées avec un tamis cellulaire 40µm puis après rinçage avec la solution 1 elles sont stockées dans une flasque de 75cm² avec 20mL de solution 4+.

Étape 2 : La flasque est gardée pendant 24h à l'incubateur à 37°C et 5% de CO².

Étape 3 : Changer le milieu tous les jours. Chaque capsule contient de 1 à 10 fibroblastes à la formation des capsules. Le virus de reprogrammation a une efficacité de transformation de l'ordre de 0.2%. La majorité des capsules ne contiendront donc pas ou très peu de cellules reprogrammées. Les cystes commencent à se former après 15 à 40 jours. Les fibroblastes ont une forme allongée et ne forment pas de cystes. Ainsi, tous les cystes qui se forment sont formés d'IPS.

## Revendications

1. Microcompartiment cellulaire comprenant successivement, organisées autour d'une lumière :
- au moins une couche de cellules pluripotentes humaines ;
- une couche de matrice extracellulaire ;
- une couche externe en hydrogel,
dans lequel ledit microcompartiment est clos.

2. Microcompartiment cellulaire selon la revendication 1, dans lequel la couche externe comprend de l'alginate.

3. Microcompartiment cellulaire selon l'une des revendications précédentes, dans lequel ledit microcompartiment a une forme sphérique ou allongée.

4. Microcompartiment cellulaire selon l'une des revendications précédentes, dans lequel ledit microcompartiment a un diamètre ou une plus petite dimension comprise entre 10 µm et 1 mm, préférentiellement entre 50 µm et 500 µm, plus préférentiellement inférieure à 500 µm, encore plus préférentiellement inférieure à 400 µm.

5. Microcompartiment cellulaire selon l'une des revendications précédentes, dans lequel la densité cellulaire est comprise entre une et plusieurs milliers de cellules, préférentiellement 50 à 1000 cellules par microcompartiments.

6. Procédé de préparation d'un microcompartiment cellulaire selon l'une des revendications 1 à 5, comprenant les étapes consistant à :
(a) incuber des cellules souches pluripotentes humaines dans un milieu de culture contenant un inhibiteur des voies RHO/ROCK ;
(b) mélanger les cellules souches pluripotentes issues de l'étape (a) avec une matrice extracellulaire ;
(c) encapsuler le mélange de l'étape (b) dans une couche d'hydrogel ;
(d) cultiver les capsules obtenues à l'étape (c) dans un milieu de culture contenant un inhibiteur des voies RHO/ROCK ;
(e) rincer les capsules issues de l'étape (d), de manière à éliminer l'inhibiteur des voies RHO/ROCK ;
(f) cultiver les capsules issues de l'étape (e) pendant 3 à 20 jours, préférentiellement 5 à 10 jours, et optionnellement récupérer les microcompartiments cellulaires obtenus.

7. Procédé de préparation d'un microcompartiment selon la revendication 6, comprenant une étape intermédiaire consistant à
(a') dissocier les cellules souches pluripotentes issues de l'étape (a) avant l'étape (b), préférentiellement au moyen d'un réactif exempt d'enzyme.

8. Procédé de préparation d'un microcompartiment cellulaire selon l'une des revendications 1 à 6, comprenant les étapes consistant à
(a) mélanger des cellules différenciées humaines avec une matrice extracellulaire et des agents de reprogrammation cellulaire ;
(b) encapsuler le mélange de l'étape (a) dans une couche d'hydrogel ;
(c) cultiver les capsules issues de l'étape (b) pendant 10 à 40 jours, et optionnellement récupérer les microcompartiments cellulaires obtenus.

9. Procédé de préparation d'un microcompartiment cellulaire selon la revendication 8, dans lequel chaque capsule issue de l'étape (b) contient entre 1 et 500 cellules différenciées.

10. Procédé de préparation d'un microcompartiment cellulaire selon l'une des revendications 6 à 10, comprenant une étape ultérieure consistant à congeler les microcompartiments cellulaires obtenus à l'étape (f) selon la revendication 6 ou à l'étape (c) selon la revendication 8.

## Patentansprüche

1. Zelluläre Mikrokammer, umfassend aufeinanderfolgend um ein Licht herum angeordnet:
- mindestens eine Schicht aus menschlichen pluripotenten Zellen;
- eine Schicht aus extrazellulärer Matrix;
- eine äußere Hydrogelschicht,
wobei die Mikrokammer geschlossen ist.

2. Zelluläre Mikrokammer nach Anspruch 1, wobei die äußere Schicht Alginat umfasst.

3. Zelluläre Mikrokammer nach einem der vorstehenden Ansprüche, wobei die Mikrokammer eine kugelförmige oder längliche Form aufweist.

4. Zelluläre Mikrokammer nach einem der vorstehenden Ansprüche, wobei die Mikrokammer einen Durchmesser oder eine kleinere Abmessung zwischen 10 µm und 1 mm, vorzugsweise zwischen 50 µm und 500 µm, mehr bevorzugt von weniger als 500 µm, noch mehr bevorzugt von weniger als 400 µm aufweist.

5. Zelluläre Mikrokammer nach einem der vorstehenden Ansprüche, wobei die Zelldichte zwischen einer und mehreren tausend Zellen, vorzugsweise 50 bis 1000 Zellen pro Mikrokammer, beträgt.

6. Verfahren zum Herstellen einer zellulären Mikrokammer nach einem der Ansprüche 1 bis 5, umfassend die Schritte bestehend aus:
(a) Inkubieren von menschlichen pluripotenten Stammzellen in einem Kulturmedium, das einen Inhibitor von RHO/ROCK-Signalwegen enthält;
(b) Mischen der pluripotenten Stammzellen aus Schritt (a) mit einer extrazellulären Matrix;
(c) Einkapseln der Mischung aus Schritt (b) in einer Hydrogelschicht;
(d) Kultivieren der in Schritt (c) erhaltenen Kapseln in einem Kulturmedium, das einen Inhibitor der RHO/ROCK-Signalwege enthält;
(e) Spülen der Kapseln aus Schritt (d), um den RHO/ROCK-Signalweg-Inhibitor zu entfernen;
(f) Kultivieren der Kapseln aus Schritt (e) 3 bis 20 Tage lang, vorzugsweise 5 bis 10 Tage, und gegebenenfalls Rückgewinnen der erhaltenen zellulären Mikrokammern.

7. Verfahren zum Herstellen einer Mikrokammer nach Anspruch 6, umfassend einen Zwischenschritt bestehend aus
(a') Dissoziieren der pluripotenten Stammzellen aus Schritt (a) vor Schritt (b), vorzugsweise mithilfe eines enzymfreien Reagenzes.

8. Verfahren zum Herstellen einer zellulären Mikrokammer nach einem der Ansprüche 1 bis 6, umfassend die Schritte bestehend aus
(a) Mischen von menschlichen differenzierten Zellen mit einer extrazellulären Matrix und Mitteln für eine Zellreprogrammierung;
(b) Einkapseln der Mischung aus Schritt (a) in einer Hydrogelschicht;
(c) Kultivieren der Kapseln aus Schritt (b) 10 bis 40 Tage lang, und gegebenenfalls Rückgewinnen der erhaltenen zellulären Mikrokammern.

9. Verfahren zum Herstellen einer zellulären Mikrokammer nach Anspruch 8, wobei jede Kapsel aus Schritt (b) zwischen 1 und 500 differenzierte Zellen enthält.

10. Verfahren zum Herstellen einer zellulären Mikrokammer nach einem der Ansprüche 6 bis 10, umfassend einen nachträglichen Schritt, der darin besteht, die in Schritt (f) nach Anspruch 6 oder in Schritt (c) nach Anspruch 8 erhaltenen zellulären Mikrokammern einzufrieren.

## Claims

1. A cellular microcompartment comprising successively, organized around a lumen:
- at least one layer of human pluripotent cells;
- an extracellular matrix layer;
- an outer layer of hydrogel,
wherein said microcompartment is closed.

2. The cellular microcompartment according to claim 1, wherein the outer layer comprises alginate.

3. The cellular microcompartment according to one of the preceding claims, wherein said microcompartment has a spherical or elongated shape.

4. The cellular microcompartment according to one of the preceding claims, wherein said microcompartment has a diameter or smaller dimension of between 10 µm and 1 mm, preferentially between 50 µm and 500 µm, more preferentially less than 500 µm, even more preferentially less than 400 µm.

5. The cellular microcompartment according to one of the preceding claims, wherein the cell density is between one and several thousand cells, preferentially 50 to 1000 cells per microcompartment.

6. A method for preparing a cellular microcompartment according to one of claims 1 to 5, comprising the following steps:
(a) incubating human pluripotent stem cells in a culture medium containing a RHO/ROCK pathway inhibitor;
(b) mixing the pluripotent stem cells from step (a) with an extracellular matrix;
(c) encapsulating the mixture from step (b) in a hydrogel layer;
(d) culturing the capsules obtained in step (c) in a culture medium containing a RHO/ROCK pathway inhibitor;
(e) rinsing the capsules from step (d) to remove the RHO/ROCK pathway inhibitor;
(f) culturing the capsules from step (e) for 3 to 20 days, preferentially for 5 to 10 days, and optionally recovering the obtained cellular microcompartments.

7. The method for preparing a microcompartment according to claim 6, comprising an intermediate step consisting in
(a') dissociating the pluripotent stem cells from step (a) before step (b), preferentially by means of an enzyme-free reagent.

8. The method for preparing a cellular microcompartment according to one of claims 1 to 6, comprising the steps of
(a) mixing human differentiated cells with an extracellular matrix and cell reprogramming agents;
(b) encapsulating the mixture from step (a) in a hydrogel layer;
(c) culturing the capsules from step (b) for 10 to 40 days, and optionally recovering the cellular microcompartments obtained.

9. The method for preparing a cellular microcompartment according to claim 8, wherein each capsule from step (b) contains between 1 and 500 differentiated cells.

10. The method for preparing a cellular microcompartment according to one of claims 6 to 10, comprising a subsequent step consisting in freezing the cellular microcompartments obtained in step (f) according to claim 6 or in step (c) according to claim 8.
